# EUROPEAN PATENT SPECIFICATION

(11) **EP 1 728 859 B1**
(45) Date of publication and mention of the grant of the patent: **04.05.2011**
(21) Application number: 05719702.2
(22) Date of filing: 01.03.2005
(51) Int. Cl.: C12N 15/09, C12N 5/10, C12Q 1/02

(54) **SEQUENCE CAPABLE OF ACCELERATING GENE EXPRESSION AT MODERATELY LOW TEMPERATURE**
ZUR BESCHLEUNIGUNG DER GENEXPRESSIONBEI EINER MÄSSIG NIEDRIGEN TEMPERATUR FÄHIGE SEQUENZ
SEQUENCE CAPABLE D'ACCELERER L'EXPRESSION GENIQUE A UNE TEMPERATURE MODEREMENT FAIBLE

(30) Priority: 23.03.2004 JP 2004084987
(43) Date of publication of application: 06.12.2006
(73) Proprietor: Fujita, Jun, Kyoto-shi, Kyoto 6060926 (JP)
(72) Inventor: Fujita, Jun, Kyoto-shi, Kyoto 6060926 (JP)
(74) Representative: Tollervey, Rebecca Marie
(86) International application number: PCT/JP2005/003385
(87) International publication number: WO 2005/090562

(56) References cited:
- WO-A-03/093500
- WO-A2-02/16655
- CAI Q. ET AL: 'An unusual group 2 LEA gene family in citrus responsive to low temperature.' PLANT MOL BIOL. vol. 29, no. 1, 1995, pages 11 - 23, XP002993546
- JARILLO J.A. ET AL: 'Two related low-temperature-inducible genes of Arabidopsis encode proteins showing high homology to 14-3-3 proteins, a family of putative kinase regulators.' PLANT MOL BIOL. vol. 25, no. 4, 1994, pages 693 - 704, XP002993547
- YIN X. ET AL: 'Isolating the promoter of a stress-induced gene encoding betaine aldehyde dehydrogenase from the halophyte Atriplex centralasiatica Iljin.' BIOCHIM BIOPHYS ACTA. vol. 1577, no. 3, 2002, pages 452 - 456, XP004383776
- NISHIYAMA H. ET AL: 'A glycine-rich RNA binding protein mediating cold-inducible suppression of mammalian cell growth.' J CELL BIOL. vol. 137, no. 4, 1997, pages 899 - 908, XP002993548
- NISHIYAMA H. ET AL: 'Cloning and characterization of human CIRP (cold-inductible RNA binding protein) cDNA and chromosomal assignment of the gene.' GENE. vol. 204, no. 1-2, 1997, pages 115 - 120, XP004100701

### SEQUENCE LISTING

<110> FUJITA, Jun
<120> Sequence Accelerating Expression of Gene at Mild Low Temperature
<130> RXT/FP6401301
<140> 05719702.2
   <141> 2005-03-01
<150> PCT/JP2005/003385
   <151> 2005-03-1
<150> JP 2004-084987
   <151> 2004-03-23
<160> 23
<170> Patent In Ver. 2.1
<210> 1
   <211> 2520
   <212> DNA
   <213> mouse
<400> 1
   ttgtcagcgc aagataggca agtgctgcat agacattcag gcaaacaccc atatacataa 60
   aattaaaata cacttttttg ttttgttctc tgtggtgtgt gtgtttaaga aggctaagga 120
   tgtagcttgg tttagccact acttagaatt ctgcagtgag gggttagggg tgtggctcaa 180
   ggatagaaat ccttccatgg catgcttgag actctggggt ctcttctaga aaatgcaggt 240
   acacattgaa aagagtggtg ttttgccatc tgtgtggtaa tgtacacctg gttggagatg 300
   ggcagaaatc agggttcaaa tggtcgttat acagcaggtt gagatttgta ggaaaactga 360
   gttgggtgca ggaaatctag agaaaatctt gacattgtga ttaatactgg gatggaaatt 420
   ttagggctga tcttatggag tgtccttggg gtccaaagaa ctgttaggca gcagttgtat 480
   tgggaggtac ttttggacta gagtaattcg gtggttttgg tttcatgagc aagaacggag 540
   aagaggggca agcaagatga ctcagtggat aaagctgctt gtggctaatc ctgagtatga 600
   tccttaggac ctacatgatg gaaggagaga ctccatctgt cttctggctt cacaaatgcg 660
   cctcagttaa ataactgtaa ttaaatatta aaagaaaaaa aagaacataa aagtgttgag 720
   tcgatgagtc aaggttggag ccatatttgg gagtctcatt gaggtagcaa aagctctttg 780
   ttccccgccg caggacaaac gggtcgggaa cgcggaagtg ggtcagctcc gcctccaaat 840
   cagctgtccc cgcctcctag tcgctagcgg gcggggggtg ggggggaaaa atgcggccag 900
   gaaagattcc agccaatcaa aacactcagc gcctccgcgc ggactagggc aaagccaacg 960
   gagtcccgcc cctccaccga ggcgcagatg ccgaatctaa ggcgtcggat tggtcagcgc 1020
   ggcgtggtgg gcgggttaag gggcgtggct ccaggagtgg agtatatcag gcgggactct 1080
   tgcgctcccc ctcgcactcg cgccttagga agcttgggtg tgtgtggcgc gctgtcttcc 1140
   cgctcgcgtc agggacctgc ccgactcagc ggtgagggct ctggcggcac cggctctgag 1200
   gggcccgggg cggcgggcgg cctgtgactc tcgggcgagg cggagccgca gcggcacgcc 1260
   cctgggactt gggggcgggc ccgggttgga gtccgccccg ccggcggcga ggcgggcagc 1320
   gggacactgg ggcctcacgc ttgtcggagg gcgtcctcca ccgcgcgtca tccccgggcc 1380
   gaggccgacc ttaacggtgg gctttttttt tttttttttt ttttgtaaaa tggccgccgc 1440
   gatgctttga aaagcttcgc cggcgaaatg gcgggactgc cgagtgcccg gatggaatgg 1500
   cgcagggtgc gcgcttggcg catgcgctgc tccgggggcg gggcgctcct acctccaaag 1560
   tccggttgtc cgcagatgtg gagctgtgtc gcgtgcgacc cttttactcc agctggctct 1620
   acctcggttt tctcttccct tgtggttcca cgtggggaac aaaaaagcac atgtaggctt 1680
   gaatgttgtc cttttgtgcc ccaagtttat tgcccccaag gtagactggt ttcgtggtat 1740
   agtggactta ttttccaagt tgtcggttgc caataatgac tttgtctaga taccacgccc 1800
   ctttcgctag ccacgcctct tgggccaacc tcctttctcc cttgagtcca tcgtgcaaaa 1860
   acatttctct agtccttggc tgagtaagcg aggcatccca caactggcac acctccagac 1920
   agtaccctaa aagcgtattt cagtagtgaa gtttctctgg cccaacaact ctatgctagt 1980
   gagggtaggc agtcactatc atttctgtcc ccaaccacac acatttgcaa agtgaccatg 2040
   ccattttgat cctaaccttg ggattctctg gccaggtgta atcaagacct agaatcattt 2100
   aaggttagcc tcagctgtat gacaaagtat tccatatgcc tttgggatga taaaagcaaa 2160
   aagagttccc tcttgtgtag cccagaaggg tattgaactt gctgtgcaat tgtatttggt 2220
   tttggattct gttcctttgc ctccaactcc cacaggctag gcgagatggg tatttgtttt 2280
   gaaacagtct ctcaaactga tccacagtgt cctttttctc attgatcttc ccatctcggt 2340
   gacttgaatg gctggatgag aggtgtgaaa gaaaccccac ttgaagctga ggtttcccag 2400
   taagaccatg gggtcctctc cccccacccc catgcttccc ccctagcccc ccccccccag 2460
   tttctttgtg tactcctggt tatcctggac tctctttgca gaccaggctg gcctcaaaac 2520
<210> 2
   <211> 8
   <212> DNA
   <213> mouse
   <400> 2
   tccccgcc 8
<210> 3
   <211> 30
   <212> DNA
   <213> mouse
   <400> 3
   ttccccgccg ttccccgccg ttccccgccg 30
<210> 4
   <211> 10
   <212> DNA
   <213> mouse
   <400> 4
   cggcggggaa 10
<210> 5
   <211> 30
   <212> DNA
   <213> mouse
   <400> 5
   cggcggggaa cggcggggaa cggcggggaa 30
<210> 6
   <211> 16
   <212> DNA
   <213> mouse
   <400> 6
   tccccgcctc cccgcc 16
<210> 7
   <211> 8
   <212> DNA
   <213> mouse
   <400> 7
   ggggggga 8
<210> 8
   <211> 24
   <212> DNA
   <213> mouse
   <400> 8
   gggggggagg gggggagggg ggga 24
<210>9
   <211>8
   <212> DNA
   <213> mouse
   <400> 9
   gccccgcc 8
<210> 10
   <211> 8
   <212> DNA
   <213> mouse
   <400> 10
   accccgcc 8
<210> 11
   <211> 8
   <212> DNA
   <213> mouse
   <400> 11
   tccccgtc 8
<210> 12
   <211> 8
   <212> DNA
   <213> mouse
   <400> 12
   tccccgct 8
<210> 13
   <211> 8
   <212> DNA
   <213> mouse
   <400> 13
   tccccacc 8
<210> 14
   <211> 8
   <212> DNA
   <213> mouse
   <400> 14
   tccccccc 8
<210> 15
   <211> 8
   <212> DNA
   <213> mouse
   <400> 15
   gacccgcc 8
<210> 16
   <211> 8
   <212> DNA
   <213> mouse
   <400> 16
   taaccgcc 8
<210> 17
   <211> 8
   <212> DNA
   <213> mouse
   <400> 17
   tcaacgcc 8
<210> 18
   <211> 8
   <212> DNA
   <213> mouse
   <400> 18
   tccaagcc 8
<210> 19
   <211> 8
   <212> DNA
   <213> mouse
   <400> 19
   tcccatcc 8
<210> 20
   <211> 8
   <212> DNA
   <213> mouse
   <400> 20
   tcccctac 8
<210> 21
   <211> 8
   <212> DNA
   <213> mouse
   <400> 21
   tccccgaa 8
<210> 22
   <211> 8
   <212> DNA
   <213> mouse
   <400> 22
   ggcgggga 8
<210> 23
   <211> 92
   <212> DNA
   <213> mouse
   <400> 23
   actcgcgcct taggaagctt gggtgtgtgt ggcgcgctgt cttcccgctc gcgtcaggga 60
   cctgcccgac tcagcggctg ccatggcatc ag                               92

## Description

### Technical Field

The present invention relates to mild low temperature response elements accelerating the transcription or the translation of a gene at mild low temperatures.

### Background Art

Various pharmaceuticals and assay reagents such as monoclonal antibodies, growth factors and clotting factors have been produced by biotechnology.

Generally, at a low temperature of 10-15°C, correct folding of heterologous recombinant protein is promoted and degradation of the protein by proteases is inhibited (Baneyx, F., In vitro folding of recombinant proteins in Escherichia coli. In Manual of industrial microbiology and biotechnology, 2nd ed., (Demain, A. L. Davis,J.E.,Altas, R.M., et al., Eds.) ASM Press, Washingtonn D. C., pp.551-1999). Accordingly, when recombinant proteins are produced by bacteria, cultivation at low temperatures below 20°C is effected, instead of at 37°C. In this case, since protein synthesis is generally lowered at low temperature, possible low yield of a desired protein may occur. This problem is solved by using the promoter of cold shock protein CspA gene and 5' untranslated region (UTR) of CspA mRNA in an expression vector of a desired protein (Baneyx, F. and Mujacic, M., Cold-inducible promoters for heterologous protein expression, Methods in Molecular Biology, 205, 1-18, 2001).

However, posttranslational modification in bacteria is significantly different from that in mammalian cells. Especially for pharmaceuticals, glycosylation state is important for therapeutic effects. Therefore, mammalian cells, particularly Chinese hamster cell lines, are used for producing protein pharmaceuticals. It is necessary to increase protein yield in order to reduce production cost with the cell cultivation. When usual cultivation at 37°C is effected, there is a disadvantage that, since cells die after rapid growth, the term during which cells survive and produce the desired protein is short. Therefore, an attempt to restrain cell growth and have cells survive for a longer time period is effected by the addition of cell growth inhibitors, the expression of cell-cycle inhibitory gene p27, the cultivation at mild low temperatures (30-32°C) , etc. (see Schatz SM et al., Biotechnol. Bioeng, 2003, Nov. 433-438).

Among them, the cultivation at mild low temperature has the characteristics as follows:
1) Cells survive and produce recombinant proteins for a longer time period than the cultivation at 37°C;
2) The activity of proteolytic enzyme is lowered to reduce the degradation of desired proteins;
3) Glycosylation and isoprotein patterns are similar to those cultivated at 37°C, and sialylation is rather better than that cultivated at 37°C;
4) A possible improvement in correct folding of desired proteins as in bacteria (Kaufmann H, Mazur X, Marone R, Bailey JE, Fussengger M., Biotech Bioeng., 2001 Mar. 20, 72(6):592-602; Yoon SK, Song JY, Lee GM., Biotech Bioeng., 2003 May 5, 82(3):289-98).

In regard to crucial yield of recombinant protein, since cell survive terms is extended, total yield is often increased. However, the specific productivity of protein per cell per unit time varies for unknown reasons, possibly depending on kinds of cell, kinds of the desired protein, and others. There are many cases in which the specific productivity is rather lower than that at 37°C, which is problematic (Kaufmann H, Mazur X, Marone R, Bailey JE, Fussenegger M., Biotech Bioeng., 2001 Mar. 20, 72(6):592-602; Yoon SK, Song JY, Lee GM., Biotech Bioeng., 2003 May. 5, 82(3):289-98). Since gene expression control mechanism is different from that in bacteria, cold shock protein CspA promoter and 5' untranslated region (UTR) of CspA mRNA in bacteria cannot be used in mammalian cells.

It is an object of the present invention to provide a method for increasing protein production and total yield per cell per unit time at low temperature.

### Disclosure of Invention

The inventor of the present application previously found and reported the cold shock proteins Cirp (cold inducible RNA-binding protein) the genomic DNA sequence of which is shown as SEQ ID No:1, and Rbm3 (RNA-binding motif protein 3) (Fujita J., Cold shock response in mammalian cells, J. Mol Microbiol. Biotechnol., 1999 Nov,1(2):243-55).

Now, the present invention has been made based on that the gene expression control sequences capable of accelerating transcription are found on 5' side to the promoter of these protein genes.

The present invention provides in a first aspect use of a mild low temperature transcription control element to accelerate the in vitro transcription or translation of a gene at mild low temperatures, wherein said mild low temperature transcription control element consists of a nucleotide sequence selected from tcccc gcc(SEQ ID NO:2), ggggg gga (SEQ ID NO:7), gcccc gcc (SEQ ID NO:9), acccc gcc (SEQ ID NO:10), tcccc gtc (SEQ ID NO: 11), tcccc gct (SEQ ID NO:12) and tcccc gaa (SEQ ID NO:21), or the complement thereof.

The term "mild low temperature" herein refers to a temperature of 15-36°C, preferably a temperature of 30-34°C and most preferably a temperature of 32°C. The term "mild low temperature transcription control element" refers to a nucleotide sequence capable of accelerating the transcription of a gene specifically at mild low temperatures. The mild low temperature transcription control element of the present invention functions as a mild low temperature specific enhancer in the transcription of a gene.

The mild low temperature transcription control element of the present invention can exhibit its function when it is used alone. However, it is preferable to use it in connection of 2-50, preferably 2-10 of the same or different elements. When the elements are used in connection, the same or different elements may be directly connected, or may be connected via any number and kind of nucleotides. The connected mild low temperature transcription control elements are referred to as "mild low temperature transcription control enhancer" in the present specification. The mild low temperature transcription control element and the mild low temperature transcription control enhancer of the present invention have no relation to the distance from a promoter.

The present invention relates to a vector comprising the mild low temperature transcription control element or the mild low temperature transcription control enhancer described above.

The present invention relates to mammalian cells transformed by the vector.

The present application describes a method for screening a mild low temperature transcription control element comprising:
1) providing a gene capable of accelerating transcription at a mild low temperature, particularly preferably at a cultivation temperature of 32°C;
2) cloning a part of the gene ranging from the 1.5-kilobase 5' to transcription initiation site to the 3' end of the first exon of the gene;
3) inserting the cloned nucleotide sequence with an appropriate promoter into a vector expressing a reporter gene;
4) transforming mammalian cells with the said vector;
5) cultivating the cells at a mild low temperature or 37°C to express the reporter gene to identify the nucleotide sequence capable of accelerating the transcription of the reporter gene at a mild low temperature; and
6) shortening the nucleotide sequence to be inserted into the vector for the nucleotide sequence capable of accelerating the transcription of the reporter gene to identify the nucleotide sequence necessary for accelerating the transcription of the reporter gene at the mild low temperature.

The present invention relates to a method for improving a mild low temperature transcription control element consisting of the sequence tccccgcc or ggggggga comprising:
1) synthesizing a mutated nucleotide sequence in which one or two nucleotides in a mild low temperature transcription control element are substituted with other nucleotides;
2) incorporating the mutated nucleotide sequence with an appropriate promoter into a vector expressing a reporter gene;
3) transforming mammalian cells with the said vector;
4) cultivating the cells at a mild low temperature or 37°C to express the reporter gene to identify the nucleotide sequence capable of accelerating the transcription of the reporter gene at the mild low temperature.

### Effect of the Present Invention

The present invention can accelerates the transcription and the translation of a gene at mild low temperatures.

### Brief Description of Drawings

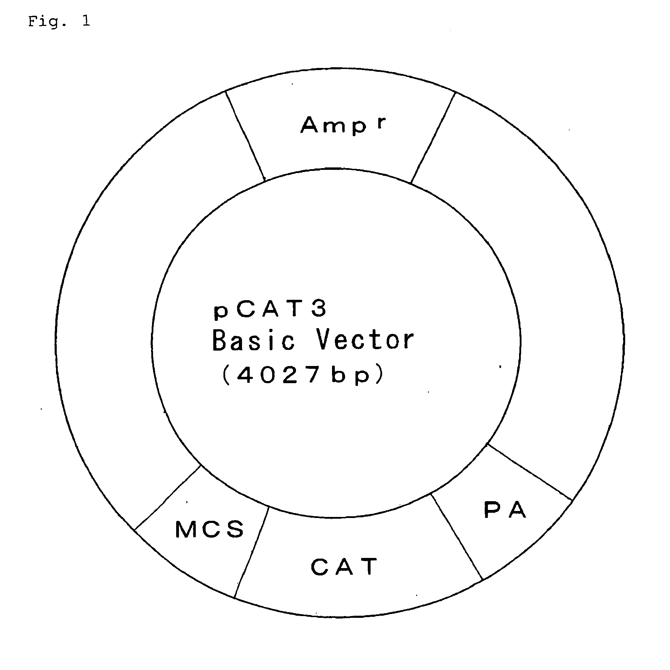

Fig. 1 is a schematic view representing pCAT3 basic vector. MCS represents a multi-cloning site. CAT represents a coding sequence of chloramphenicol acetyltransferase. PA represents a polyadenylation signal derived from SV40 virus. Amp^{r} represents ampicillin resistance gene.

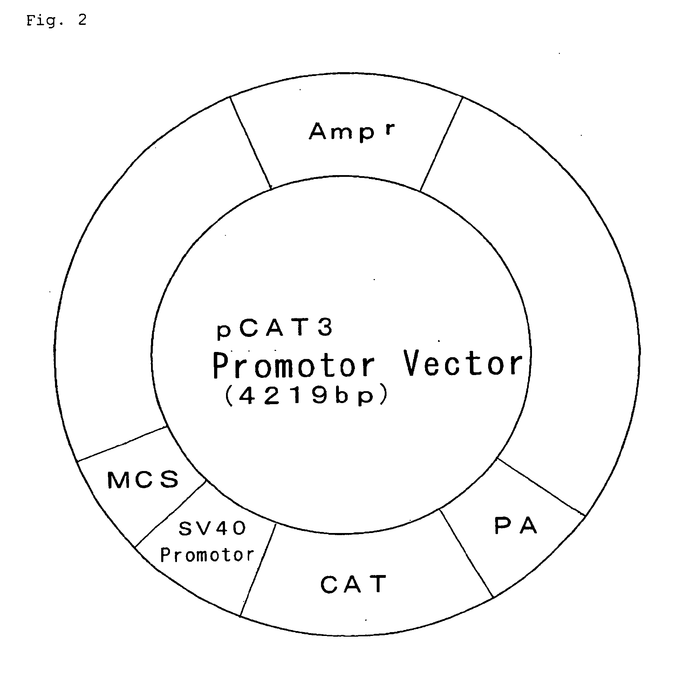

Fig. 2 is a schematic view representing pCAT3 promoter vector. MCS represents a multi-cloning site. SV40 promoter represents a promoter derived from SV40 virus. CAT represents a coding sequence of chloramphenicol acetyltransferase. PA represents a polyadenylation signal derived from SV40 virus. Amp^{r} represents an ampicillin resistance gene.

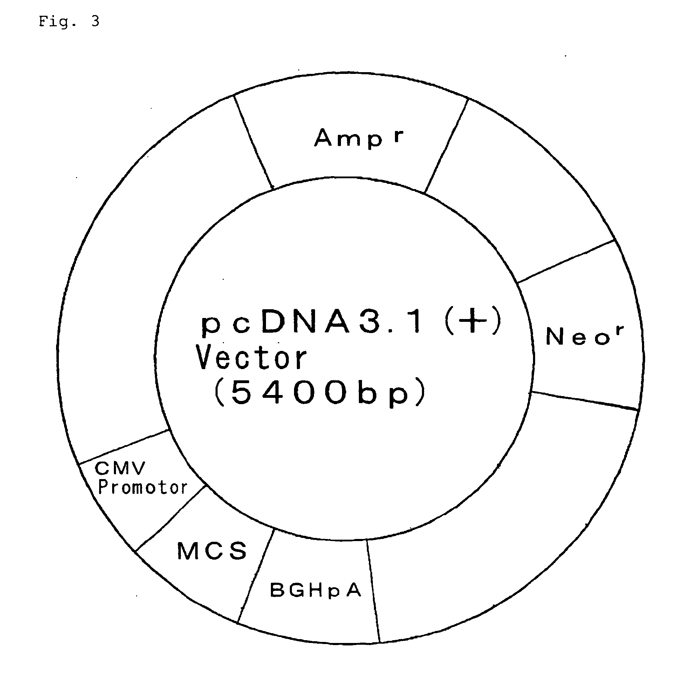

Fig. 3 is a schematic view representing pcDNA 3. 1 (+) vector (Invitrogen). MCS represents a multi-cloning site. Firefly luciferase encoding sequence is inserted between EcoRV and XbaI of the multi-cloning site. Complementary DNA (cDNA) for 5' untranslated region (UTR) of Cirp mRNA is inserted between KpnI and BamHI of the multi-cloning site

### Best Mode for Carrying Out the Invention

Examples of the mild low temperature transcription control element of the present invention include, but is not limited to, tcccc gcc (SEQ ID NO:2), gcccc gcc (SEQ ID NO:9), acccc gcc (SEQ ID NO:10), tcccc gtc (SEQ ID NO:11), tcccc gct (SEQ ID NO:12), tcccc gaa (SEQ ID NO:21), ggcgg gga (SEQ ID NO:22) (the complement of SEQ ID NO: 2) and ggggg gga (SEQ ID NO:7)

### (i) Preparation of mild low temperature response element

The mild low temperature transcription control element or the mild low temperature transcription control enhancer of the present invention, and the mild low temperature translation control element described herein can be prepared, for example, via a solid synthesis method by phosphoroamidide method or via a solid synthesis method by hydrogen phosphonate method, using four kinds of deoxyribonucleotide with protective groups (see, for example, Oligonucleotide Synthesis: A Practical Approach, IRL Press, 35-81,83-115 (1984); J. Org. Chem., 49, 2139 (1984); Tetrahedron Lett., 27, 469 (1986); Tetrahedron Lett.,22, 1859-1862; Tetrahedron Lett., 21, 719-722; J. Am. Chem. Soc., 103, 3185-3191 (1981). Nowadays they can be easily synthesized with commercially available DNA synthesizers (for example, manufactured by Applied Biosystem).

### (ii) Promoter to be used

Prokaryote promoters, eukaryote promoters and virus promoters can be used as promoters. Mammalian promoters and virus promoters are preferred. Examples of preferred promoters include mouse thymidine kinase promoter (TK), cytomegalovirus (CMV) promoter and SV40 promoter.

### (iii) Coding Sequence

Genes to be expressed are not limited, and the nucleotide sequence encoding any protein can be used.

### (iv) Vector to be used

The mild low temperature transcription control element or the mild low temperature transcription control enhancer of the present invention, a promoter and the coding sequence encoding a protein to be expressed are linked together and inserted into a vector. The expression vector for prokaryote cell and eukaryote cell can be used. Examples of preferred vector include, but not limited to, pCMV (Invitrogen), pCAT3 promoter vector (Promega) and pSV2-neo vector.

### (v) Host cell to be used

Host cells are transfected with the vector. It is preferred to use mammalian cells as host cell. Examples of preferred mammalian cell are human cell lines such as 293, 293T, U-20S, Huh-7, HeLa cell; monkey cell lines such as COS-7 and Vero cell; mouse cell lines such as NIH/3T3, Balb/3T3, B-6, Hepal-6 and Chinese hamster cell lines such as CHO.

The methods for the transfection include calcium phosphate method, lipofection method and electroporation method. For example, in the calcium phosphate method, calcium chloride solution containing DNA (vector) is added dropwise to phosphoric acid solution with stirring. Refined precipitation containing DNA-calcium phosphate is formed. When the solution is added to host cell, the precipitation of DNA-calcium phosphate enters into host cells via phagocytosis so that DNA is transfected into cells.

### (vi) Cultivation condition

The host cell is cultivated at a mild low temperature. It is unnecessary to use special medium as culture medium. Usual medium for mammal cell cultivation can be used. Typical example of the medium is Dulbecco's modified Eagle's medium (D-MEM) supplemented with 10% fetal calf serum (FCS) (Invitrogen).

The present application describes a method for screening a mild low temperature transcription control element comprising:
1) providing a gene capable of accelerating transcription at a mild low temperature, particularly preferably at a cultivation temperature of 32°C;
2) cloning a part of the gene ranging from the 1.5-kilobase 5' to transcription initiation site to the 3' end of the first exon of the gene;
3) incorporating the cloned nucleotide sequence with an appropriate promoter into a vector expressing a reporter gene;
4) transforming mammalian cells with the said vector;
5) cultivating the cells at a mild low temperature or 37°C to express the reporter gene to identify the nucleotide sequence capable of accelerating the transcription of the reporter gene at a mild low temperature; and
6) shortening the nucleotide sequence to be inserted into the vector for the nucleotide sequence capable of accelerating the transcription of the reporter gene to identify the nucleotide sequence necessary for accelerating the transcription of the reporter gene at the mild low temperature.

The gene capable of accelerating transcription at mild low temperature can be obtained by cultivating mammalian cells at 37°C and at mild low temperature, preferably at 32°C and applying DNA chip (microaray) or cDNA subtraction method (see, for example, Moody, D.E., Genomic techniques: An overview of methods for study of gene expression, J. Anim. Sci., 79(E. Suppl.): E128-E135. 2001) to the RNA extracted therefrom.

Prokaryote promoters, eukaryote promoters and virus promoters can be used as promoters. Mammalian promoters and virus promoters are preferred. Examples of preferred promoters include, but is not limited to, mouse thymidine kinase promoter (TK), cytomegalovirus (CMV) promoter and SV40 promoter.

Reporter gene is a gene encoding protein capable of being easily detected, and include, but is not limited to, luciferase gene and chloramphenicol acetyltransferase gene. Vectors for prokaryote cell and eukaryote cell can be used. Examples of preferred vector include, but is not limited to, pCMV (Invitrogen), pCAT3 promoter vector (Promega) and pSV2-neo vector.

Host cells are transfected with the vector. It is preferred to use mammalian cells as host cell. Examples of preferred mammalian cell are human cell lines such as 293, 293T, U-2OS, Huh-7, HeLa cell; monkey cell lines such as COS-7 and Vero cell; mouse cell lines such as NIH/3T3, Balb/3T3, B-6, Hepal-6 and Chinese hamster such as CHO

The methods for the transfection include calcium phosphate method, lipofection method and electroporation method. For example, in the calcium phosphate method, calcium chloride solution containing DNA (vector) is added dropwise to phosphoric acid solution with stirring. Refined precipitation containing DNA-calcium phosphate is formed. When the solution is added to host cell, the precipitation of DNA-calcium phosphate enters into host cells via phagocytosis so that DNA is transfected into cells.

It is unnecessary to use special medium as culture medium. Usual medium for mammalian cell cultivation can be used. A typical example of medium is Dulbecco's modified Eagle's medium (D-MEM) supplemented with 10% fetal calf serum (FCS)(Invitrogen).

If the ratio of the amount of the reporter gene product expressed at a mild low temperature, preferably at 32°C, to that at 37°C is more than 1, the nucleotide sequence can be an enhancer capable of accelerating the transcription of gene specifically at mild low temperature. The nucleotide sequence capable of accelerating the transcription of the gene at a mild low temperature is shortened to obtain a mild low temperature transcription control element of the present invention. That it is an enhancer can be confirmed by confirming that the nucleotide sequence complementary to the mild low temperature transcription control element can also accelerate the transcription of gene at a mild low temperature.

The present invention relates to a method for improving a mild low temperature transcription control element consisting of the sequence tccccgcc or ggggggga comprising:
1) synthesizing a mutated nucleotide sequence in which one or two nucleotides in a mild low temperature transcription control element are substituted with other nucleotide;
2) incorporating the mutated nucleotide sequence with an appropriate promoter into a vector expressing a reporter gene;
3) transforming mammalian cells with the said vector;
4) cultivating the cells at a mild low temperature or 37°C to express the reporter gene to identify the nucleotide sequence capable of accelerating the transcription of the reporter gene at the mild low temperature.

The mutated nucleotide in which one or two nucleotides in the mild low temperature transcription control element are substituted with other nucleotides can be synthesized as described above.

Prokaryote promoters, eukaryote promoters and virus promoters can be used as promoters. Mammalian promoters and virus promoters are preferred. Examples of preferred promoters include mouse thymidine kinase promoter (TK), cytomegalovirus (CMV) promoter and SV40 promoter.

Reporter gene is a gene encoding protein capable of being easily detected, and includes luciferase gene chloramphenicol acetyltransferase gene. Vector for prokaryote cell and eukaryote cell can be used. Examples of preferred vector include, but is not limited to, pCMV (Invitrogen), pCAT3 promoter vector (Promega) and pSV2-neo vector.

Host cells are transfected with the vector. It is preferred to use mammalian cells as host cell. Examples of preferred mammalian cell are human cell lines such as 293, 293T, U-2OS Huh-7, HeLa cell; monkey cell lines such as COS-7 and Vero cell; mouse cell lines such as NIH/3T3, Balb/3T3, B-6, Hepal-6 and Chinese hamster such as CHO.

The methods for the transfection include calcium phosphate method, lipofection method and electroporation method. For example, in the calcium phosphate method, calcium chloride solution containing DNA (vector) is added dropwise to phosphoric acid solution with stirring. Refined precipitation containing DNA-calcium phosphate is formed. When the solution is added to host cell, the precipitation of DNA-calcium phosphate enters into host cells via phagocytosis so that DNA is transfected into cell.

It is unnecessary to use special medium as culture medium. Usual medium for mammalian cell cultivation can be used. Typical example of the medium is Dulbecco's modified Eagle's medium (D-MEM) supplemented with 10% fetal calf serum (FCS)(Invitrogen).

If the ratio of the amount of the reporter gene product expressed at a mild low temperature, preferably at 32°C, to that at 37°C is more than 1, the sequence can be an enhancer capable of accelerating the transcription of gene specifically at a mild low temperature. That the sequence is an enhancer can be confirmed by confirming that the nucleotide sequence complementary to the mild low temperature transcription control element of the present invention also accelerates the transcription of gene at a mild low temperature.

### Example

### Example 1

Assuming "a" of "actcgc · · · " of the transcription start site of Cirp gene to be +1, the Cirp gene spanning the nucleotides -970 (970 nucleotides 5' to the transcription start site) to +56 (within the first exon), and fragments thereof deleted on 5' side (see Table 1) were inserted into the multi-cloning site of pCAT3-basic vector (Promega). The vector has chloramphenicol acetyltransferase (CAT) gene as the reporter gene.

Then 2.5-5.0µg/35-mm dish of the plasmids were co-transfected with 0.5-1.0µg/35-mm dish of CDM8-LacZ (which is β-galactosidase expression vector and used to correct for transfection efficiency) into human cell line 293.

The transfected cells were divided into two groups. One is cultivated at 32°C and the other at 37°C. Medium was D-MEM supplemented with 10% FCS.

After 36 hours, cells were recovered by centrifugation and whole cell lysate was prepared. The amount of chloramphenicol acetyltrnsferase (CAT) protein produced at each temperature was determined using CAT ELISA kit (Roche Diagnostic), and β-galactosidase activity was determined usingβ-galactosidase assay kit (Stratagene). The transcription accelerating activity of each fragment is shown in Table 1. The transcription accelerating activity was calculated by dividing CAT protein mass by β-galactosidase activity.

**Table 1**

| Transcription accelerating activity of 5' side fragment of Cirp genome | | | |
|---|---|---|---|
| Cirp genome 5'side fragment | CAT/Lac Z | | |
| | 32°C | 37°C | 32°C/37°C ratio |
| -970∼+56 | 2.007±0.005 | 1.173±0.004 | 1.711 |
| -910∼+56 | 2.804±0.009 | 1.196±0.008 | 2.345 |
| -850∼+56 | 2.518±0.007 | 1.250±0.010 | 2.015 |
| -790∼+56 | 2.014±0.003 | 1.211±0.009 | 1.664 |
| -720∼+56 | 2.586±0.009 | 1.246±0.007 | 2.076 |
| -620∼+56 | 2.008±0.010 | 1.180±0.007 | 1.702 |
| -520∼+56 | 2.251±0.003 | 1.481±0.011 | 1.520 |
| -430∼+56 | 2.298±0.010 | 1.640±0.008 | 1.402 |
| -340∼+56 | 2.729±0.003 | 1.365±0.004 | 2.000 |
| -220∼+56 | 0.045±0.003 | 0.123±0.002 | 0.366 |

The data at 24 hours after transfection was similar this result.

From these data, it is considered that basic promoter for gene transcription may exist at -220 to 0 of the genomic gene and an enhancer accelerating transcription in response to 32°C may exist at -340 to -220.

### Example 2

Example 1 was repeated except that human cell line 293T, U-2 OS, or Huh-7, monkey cell line COS-7, or mouse cell line NIH/3T3, Balb/3T3, B-6 or Hepal-6 was used in place of human 293 as host cells, and similar results were obtained.

### Example 3

Various deletion mutants derived from the nucleotides -340 to -240 of the Cirp gene were prepared and inserted into the multi-cloning site of pCAT3 promoter vector (Promega) (Fig. 2). This vector has the promoter of SV40. As in Example 1, after the gene transfer by DNA transfection with calcium phosphate method using human 293 cells, transcription accelerating activity was measured at 32°C. pCAT 3 promoter vector containing no insert was used as a negative control (mock).

Data at 36 hours after transfection are shown. The transcription accelerating activity was calculated by dividing CAT protein mass by β-galactosidase activity (Table 2).

**Table 2**

| Transcription accelerating activity of 5' side fragment of Cirp genome | | | |
|---|---|---|---|
| Mutant with deletion in -340 to -220 range | CAT/Lac Z | | |
| | 32°C | 37°C | 32°C/37°C ratio |
| mock | 0.226±0.012 | 0.757±0.010 | 0.299 |
| -340∼-220 | 2.629±0.008 | 0.592±0.004 | 9.441 |
| -340∼-290 | 4.064±0.015 | 0.654±0.010 | 6.215 |
| -290∼-220 | 2.419±0.008 | 0.549±0.008 | 4.406 |
| -310∼-260 | 2.089±0.011 | 0.540±0.003 | 3.869 |
| -390∼-310 | 7.697±0.008 | 9.211±0.031 | 1.828 |
| -310∼-290 | 8.234±0.015 | 2.064±0.017 | 3.990 |
| -290∼-260 | 10.217±0.011 | 6.281±0.030 | 1.627 |
| -260∼-220 | 9.681±0.011 | 3.849±0.011 | 2.519 |

It is considered from these results that there are enhancers in 2 genomic fragments, -310 to -290, and -260 to -220, which accelerate transcription in response to 32°C. Comparing the nucleotide sequence within the two fragments, common sequence of tcccc gcc (SEQ ID NO:2) has been found.

### Example 4

Three molecules of ten nucleotides containing tcccc gcc found above were connected in series to form a sequence ttccc cgccg ttccc cgccg ttccc cgccg (SEQ ID NO: 3). The sequence was inserted into the multi-cloning site of pCAT3 promoter vector as in Example 3. As a negative control, pCAT3 promoter vector containing no insert was used (mock). As positive controls, pCAT3 promoter vector containing Cirp genome from -340 to -220 as an enhancer and pCAT3 promoter vector containing SV 40 enhancer were used. The result obtained at 24 hours after transfection into 293 cells is shown in Table 3.

**Table 3**

| Enhancer | CAT/Lac Z | | |
|---|---|---|---|
| | 32°C | 37°C | 32°C/37°C ratio |
| mock | 0.220±0.003 | 0.677±0.012 | 0.325 |
| SV40 | 0.908±0.003 | 1.371±0.010 | 0.662 |
| ttccccgccg | 6.622±0.020 | 1.603±0.009 | 4.132 |
| ttccccgccg | | | |
| ttccccgccg | | | |
| -320 - -220 | 6.991±0.031 | 1.461±0.017 | 4.786 |

### Example 5

If a sequence is an enhancer, the reverse-directed sequence should be also effective. Three molecules of ten nucleotides containing cggcg gggaa (SEQ ID NO:4) which is a reverse directed sequence of a sequence comprising tcccc gcc, were connected in series to form a sequence cggcg gggaa cggcg gggaa cggcg gggaa (SEQ ID NO:5). The sequence was inserted into the multi-cloning site of pCAT3 promoter vector to carry out an experiment similar to Example 4.

In this case, the ratio of 32°C/37°C was 3.83, demonstrating that the sequence is a mild low temperature transcription control enhancer.

### Example 6

A sequence tcccc gcctc cccgc c(SEQ ID NO:6)obtained by connecting two molecules of 8 nucleotides tcccc gcc(SEQ ID NO:2) found in mouse Cirp genome in series was inserted into the multi-cloning site of pCAT3 promoter vector and an experiment similar to Example 4 was carried out. In this case, the ratio of 32°C/37°C was 2.51, demonstrating that the sequence is a mild low temperature transcription control enhancer.

### Example 7

Three molecules of 8 nucleotides ggggg gga (SEQ ID NO:7), the sequence of which is found about 260 nucleotides 5' to the transcription start site of human RBM genome (Gen Bank NT_011568), were connected in series to form a sequence ggggg ggagg ggggg agggg ggga (SEQ ID NO:8). The sequence was inserted into the multi-cloning site of pCAT3 promoter vector and an experiment similar to Examples-was carried out.

In this case, the ratio of 32°C/37°C was 2.52, demonstrating that the sequence is a mild low temperature transcription control enhancer.

### Example 8

Three molecules of 10 nucleotides containing one or two base-substitutions (see the table below) in the sequence tcccc gcc (SEQ ID NO:2) found above were connected in series to form a sequence. The sequence was inserted into the same vector as in Example 4, and 293 cells were transfected by the vector. After 24 hours, CAT/LacZ was measured and the mild low transcription accelerating (enhancer) activity of respective mutants was shown in the table below, defining the ratio of 32°C /37°C as 1 when pCAT3 promoter vector containing no insert was used.

**Table 4**

| Low temperature transcription accelerating (enhancer) activity of mutant with one base-substitution | | |
|---|---|---|
| Element | nucleotide sequence | Enhancer activity |
| wild type | tcccc gcc (SEQ ID NO: 2) | 3.89 |
| mutant lg | Gcccc gcc (SEQ ID NO:9) | 3.60 |
| mutant la | Acccc gcc (SEQ ID NO:10) | 3.74 |
| mutant 7t | tcccc gTc (SEQ ID NO:11) | 3.63 |
| mutant 8t | tcccc gcT (SEQ ID NO:12) | 4.08 |
| mutant 6a | tcccc Acc (SEQ ID NO:13) | 2.14 |
| mutant 6c | tcccc Ccc (SEQ ID NO:14) | 2.27 |
| control | | 1.00 |

**Table 5**

| Low temperature transcription accelerating (enhancer) activity of mutant with 2 base-substitutions | | |
|---|---|---|
| Element | nucleotide sequence | Enhancer activity |
| wild type | tcccc gcc (SEQ ID NO:2) | 3.89 |
| mutant 12 | GAccc gcc (SEQ ID NO:15) | 1.76 |
| mutant 23 | tAAcc gcc (SEQ ID NO:16) | 1.07 |
| mutant 34 | tcAAc gcc (SEQ ID NO:17) | 1.47 |
| mutant 45 | tccAA gcc (SEQ ID NO:18) | 0.35 |
| mutant 56 | tcccA Tcc (SEQ ID NO:19) | 1.07 |
| mutant 67 | tcccc TAc (SEQ ID NO:20) | 1.66 |
| mutant 78 | tcccc gAA (SEQ ID NO:21) | 3.69 |
| control | | 1.00 |

The mutants with a replacement of the first base by G or A, a replacement of the seventh base by T, and a replacement of the eighth base by T exhibit the similar transcription accelerating activity to wild type sequence. Although the replacement of the sixth base by A or C lowers the activity compared with wild type sequence, they show significant activities. The mutant 78, having replacements of the seventh and eighth bases by AA, has the same degree of transcription accelerating activity as the wild type.

Accordingly, a sequence of tcccc gcc is a sequence showing transcription accelerating activity, and the sequence:
t(or g or a)ccccg(or a or c)c(or a or t)c(or a or t) can have transcription accelerating activity.

Since they are enhancers, the sequence complementary to them, for example, ggcgg gga (SEQ ID NO:22) complementary to tcccc gcc (SEQ ID NO:2) can also be used.

### Example 9

Firefly luciferase cDNA excised from pGL3-basic plasmid (Promega) was inserted into the multi-cloning site (between EcoRV and XbaI) of pcDNA3.1 (+)vector (Invitrogen) having cytomegalovirus (CMV) promoter. Then, a cDNA fragment corresponding to the +1 to +82 nucleotides ("a" of "act · · · " of mouse Cirp transcription product (mRNA) being assumed to be +1) of Cirp mRNA, actcgcgcct taggaagctt gggtgtgtgt ggcgcgctgt cttcccgctc gcgtcaggga cctgcccgac tcagcggctg cc (SEQ ID NO:23) was inserted into the multi-cloning site (between KpnI and BamHI) of pcDNA3.1 (+) vector (Invitrogen). Control is a plasmid having no insert between KpnI and BamHI.

Then 2.5-5.0µg/35-mm dish of these plasmids were co-transfected with 0.5-1.0µg/35mm-dish of pRLTK plasmid (which is renila lucuferase expression vector and used to amend efficiency of each transfection) into human cell line 293T which had been cultivated for one day. The transfected cells were divided into two groups. One is cultivated at 32°C and the other at 37°C. Medium was D-MEM supplemented with 10% FCS.

After 36 hours, cells were recovered by centrifugation and whole cell lysate was prepared. The firefly and renila luciferase activities were determined using Dual-Luciferase Reporter kit (Promega). Translation accelerating activity of each fragment is shown in Table 6. The translation accelerating activity is shown as a value of firefly luciferase activity divided by renila luciferase activity.

**Table 6**

| Translation accelerating activity of 5' untranslated region (UTR) of Cirp mRNA | | | |
|---|---|---|---|
| 5' untranslated region (UTR) of Cirp mRNA | Firefly luciferase/Renila luciferase | | |
| | 32°C | 37°C | 32°C/37°C ratio |
| absence | 3.52 | 6.08 | 0.58 |
| presence | 6.02 | 7.71 | 0.78 |

The above result shows that the amount of protein produced at 32°C is increased by a factor of 1.7 when 5'UTR of Cirp mRNA is present in the vector compared with that in its absence, and that the amount is equal to the amount produced at 37°C in cells transfected with plasmids containing no 5'UTR of Cirp mRNA.

## Claims

1. Use of a mild low temperature transcription control element to accelerate the in vitro transcription or translation of a gene at mild low temperatures, wherein said mild low temperature transcription control element consists of a nucleotide sequence selected from tcccc gcc(SEQ ID NO:2), ggggg gga (SEQ ID NO:7), gcccc gcc (SEQ ID NO:9), acccc gcc (SEQ ID NO:10), tcccc gtc (SEQ ID NO: 11), tcccc gct (SEQ ID NO:12) and tcccc gaa (SEQ ID NO:21), or the complement thereof.

2. The use of claim 1, wherein identical or different mild low temperature transcription control elements consisting of a nucleotide sequence selected from tcccc gcc(SEQ ID NO:2), ggggg gga (SEQ ID NO:7), gcccc gcc (SEQ ID NO:9), acccc gcc (SEQ ID NO:10), tcccc gtc (SEQ ID NO: 11), tcccc gct (SEQ ID NO:12) and tcccc gaa (SEQ ID NO:21), or the complement thereof, are connected.

3. The use of claim 2, wherein said identical or different mild low temperature transcription control elements are connected via other nucleotide(s).

4. A vector comprising a mild low temperature transcription control element consisting of a nucleotide sequence selected from tcccc gcc(SEQ ID NO:2), ggggg gga (SEQ ID NO:7), gcccc gcc (SEQ ID NO:9), acccc gcc (SEQ ID NO:10), tcccc gtc (SEQ ID NO: 11), tcccc gct (SEQ ID NO:12) and tcccc gaa (SEQ ID NO:21), or the complement thereof, a promoter, and a coding sequence encoding a protein to be expressed.

5. The vector of claim 4, wherein identical or different mild low temperature transcription control elements consisting of a nucleotide sequence selected from tcccc gcc (SEQ ID NO:2), ggggg gga (SEQ ID NO:7), gcccc gcc (SEQ ID NO:9), acccc gcc (SEQ ID NO:10), tcccc gtc (SEQ ID NO: 11), tcccc gct (SEQ ID NO:12) and tcccc gaa (SEQ ID NO:21), or the complement thereof, are connected.

6. The vector of claim 5, wherein said identical or different mild low temperature transcription control elements are connected via other nucleotide(s).

7. The vector of any one of claims 4 to 6, which is a mammalian vector.

8. A mammalian cell transformed with the vector of any one of claims 4 to 7.

9. A method of producing a vector able to express a protein of interest at mild low temperatures, the method comprising linking together and inserting into a vector a mild low temperature transcription control element consisting of a nucleotide sequence selected from tcccc gcc(SEQ ID NO:2), ggggg gga (SEQ ID NO:7), gcccc gcc (SEQ ID NO:9), acccc gcc (SEQ ID NO:10), tcccc gtc (SEQ ID NO: 11), tcccc gct (SEQ ID NO:12) and tcccc gaa (SEQ ID NO:21), or the complement thereof, a promoter and the coding sequence encoding the protein to be expressed.

10. The method of claim 9, wherein the vector is a vector according to any one of claims 4 to 7.

11. A method of producing a host cell able to express a protein of interest at mild low temperatures, comprising transforming a host cell with a vector according to any one of claims 4 to 7.

12. The method of claim 11, wherein the host cell is a mammalian cell.

13. A method for improving a mild low temperature transcription control element consisting of the sequence tccccgcc or ggggggga comprising:
1) synthesizing a mutated nucleotide sequence in which one or two nucleotides in the mild low temperature transcription control element are substituted with other nucleotide;
2) incorporating the mutated nucleotide sequence with an appropriate promoter into a vector expressing chloramphenicol actyltransferase (CAT) as a reporter gene;
3) transforming mammalian cells with the said vector;
4) cultivating the cells at a mild low temperature and 37°Crespectively, to express the reporter gene to identify the nucleotide sequence necessary for accelerating the transcription of the reporter gene at a mild low temperature.

## Patentansprüche

1. Verwendung eines Transkriptionskontrollelements für schonend-niedrige Temperaturen zur Beschleunigung der In-vitro-Transkription oder -Translation eines Gens bei schonend-niedrigen Temperaturen, wobei das Transkriptionskontrollelement für schonend-niedrige Temperaturen aus einer Nucleotidsequenz besteht, die aus tcccc gcc (Seq.-ID Nr. 2), ggggg gga (Seq.-ID Nr. 7), gcccc gcc (Seq.-ID Nr. 9), acccc gcc (Seq.-ID Nr. 10), tcccc gtc (Seq.-ID Nr. 11), tcccc gct (Seq.-ID Nr. 12) und tcccc gaa (Seq.-ID Nr. 21) und deren Komplementen ausgewählt ist.

2. Verwendung nach Anspruch 1, worin identische oder unterschiedliche Transkriptionskontrollelemente für schonend-niedrige Temperaturen, die aus einer Nucleotidsequenz bestehen, die aus tcccc gcc (Seq.-ID Nr. 2), ggggg gga (Seq.-ID Nr. 7), gcccc gcc (Seq.-ID Nr. 9), acccc gcc (Seq.-ID Nr. 10), tcccc gtc (Seq.-ID Nr. 11), tcccc gct (Seq.-ID Nr. 12) und tcccc gaa (Seq.-ID Nr. 21) und deren Komplementen ausgewählt ist, verbunden werden.

3. Verwendung nach Anspruch 2, worin identische oder unterschiedliche Transkriptionskontrollelemente für schonend-niedrige Temperaturen über ein oder mehrere andere Nucleotide verbunden werden.

4. Vektor, der ein Transkriptionskontrollelement für schonend-niedrige Temperaturen, das aus einer Nucleotidsequenz besteht, die aus tcccc gcc (Seq.-ID Nr. 2), ggggg gga (Seq.-ID Nr. 7), gcccc gcc (Seq.-ID Nr. 9), acccc gcc (Seq.-ID Nr. 10), tcccc gtc (Seq.-ID Nr. 11), tcccc gct (Seq.-ID Nr. 12) und tcccc gaa (Seq.-ID Nr. 21) und deren Komplementen ausgewählt ist, einen Promotor und eine kodierende Sequenz, die für ein Protein kodiert, das exprimiert werden soll, umfasst.

5. Vektor nach Anspruch 4, worin identische oder unterschiedliche Transkriptionskontrollelemente für schonend-niedrige Temperaturen, die aus einer Nucleotidsequenz bestehen, die aus tcccc gcc (Seq.-ID Nr. 2), ggggg gga (Seq.-ID Nr. 7), gcccc gcc (Seq.-ID Nr. 9), acccc gcc (Seq.-ID Nr. 10), tcccc gtc (Seq.-ID Nr. 11), tcccc gct (Seq.-ID Nr. 12) und tcccc gaa (Seq.-ID Nr. 21) und deren Komplementen ausgewählt ist, verbunden sind.

6. Vektor nach Anspruch 5, worin identische oder unterschiedliche Transkriptionskontrollelemente für schonend-niedrige Temperaturen über ein oder mehrere andere Nucleotide verbunden sind.

7. Vektor nach einem der Ansprüche 4 bis 6, der ein Säugetiervektor ist.

8. Säugetierzelle, die mit einem Vektor nach einem der Ansprüche 4 bis 7 transformiert ist.

9. Verfahren zur Herstellung eines Vektors, der in der Lage ist, ein Protein von Interesse bei schonend-niedrigen Temperaturen zu exprimieren, wobei das Verfahren Folgendes umfasst: das Verbinden eines Transkriptionskontrollelements für schonend-niedrige Temperaturen, das aus einer Nucleotidsequenz besteht, die aus tcccc gcc (Seq.-ID Nr. 2), ggggg gga (Seq.-ID Nr. 7), gcccc gcc (Seq.-ID Nr. 9), acccc gcc (Seq.-ID Nr. 10), tcccc gtc (Seq.-ID Nr. 11), tcccc gct (Seq.-ID Nr. 12) und tcccc gaa (Seq.-ID Nr. 21) und deren Komplementen ausgewählt ist, eines Promotors und einer kodierenden Sequenz, die für das Protein kodiert, das exprimiert werden soll, und deren Insertieren in einen Vektor.

10. Verfahren nach Anspruch 9, worin der Vektor ein Vektor nach einem der Ansprüche 4 bis 7 ist.

11. Verfahren zur Herstellung einer Wirtszelle, die in der Lage ist, ein Protein von Interesse bei schonend-niedrigen Temperaturen zu exprimieren und das das Transformieren einer Wirtszelle mit einem Vektor nach einem der Ansprüche 4 bis 7 umfasst.

12. Verfahren nach Anspruch 11, worin die Wirtszelle eine Säugetierzelle ist.

13. Verfahren zur Verbesserung eines Transkriptionskontrollelements für schonend-niedrige Temperaturen, das aus der Sequenz tccccgcc oder ggggggga besteht, wobei das Verfahren Folgendes umfasst:
1) das Synthetisieren einer mutierten Nucleotidsequenz, in der ein oder zwei Nucleotide in dem Transkriptionskontrollelement für schonend-niedrige Temperaturen durch andere Nucleotide ersetzt sind;
2) das Einbauen der mutierten Nucleotidsequenz zusammen mit einem geeigneten Promotor in einen Vektor, der Chloramphenicol-Actyl-Transferase (CAT) als Reportergen exprimiert;
3) das Transformieren von Säugetierzellen mit dem Vektor; und
4) das Kultivieren der Zellen bei schonend-niedrigen Temperaturen bzw. 37 °C, um das Reportergen zu exprimieren, um die Nucleotidsequenz zu identifizieren, die zur Beschleunigung der Transkription des Reportergens bei schonend-niedrigen Temperaturen erforderlich ist.

## Revendications

1. Utilisation d'un élément de contrôle de transcription à basse température douce pour accélérer la transcription ou translation *in vitro* d'un gène à des basses températures douces, où ledit élément de contrôle de transcription à basse température douce consiste en une séquence de nucléotides sélectionnée parmi tcccc gcc(SEQ ID NO:2), ggggg gga (SEQ ID NO:7), gcccc gcc (SEQ ID NO:9), acccc gcc (SEQ ID NO:10), tcccc gtc (SEQ ID NO:9), acccc gcc (SEQ ID NO:10), tcccc gtc (SEQ ID NO:11), tcccc gct (SEQ ID NO:12) et tcccc gaa (SEQ ID NO:21), ou le complément de celle-ci.

2. Utilisation selon la revendication 1, dans laquelle des éléments de contrôle de transcription identiques ou différents à basse température douce consistant en une séquence de nucléotides sélectionnée parmi tcccc gcc (SEQ ID NO:2), ggggg gga (SEQ ID NO:7), gcccc gcc (SEQ ID NO:9), acccc gcc (SEQ ID NO:10), tcccc gtc (SEQ ID NO : 9) , acccc gcc (SEQ ID NO:10), tcccc gtc (SEQ ID NO:11), tcccc gct (SEQ ID NO:12) et tcccc gaa (SEQ ID NO:21), ou le complément de celle-ci, sont reliés.

3. Utilisation selon la revendication 2, dans laquelle lesdits éléments de contrôle de transcription identiques ou différents à basse température douce sont reliés par un autre ou par d'autres nucléotides.

4. Vecteur comprenant un élément de contrôle de transcription à basse température douce consistant en une séquence de nucléotides sélectionnée parmi tcccc gcc (SEQ ID NO:2), ggggg gga (SEQ ID NO:7), gcccc gcc (SEQ ID NO:9), acccc gcc (SEQ ID NO:10), tcccc gtc (SEQ ID NO:9), acccc gcc (SEQ ID NO:10), tcccc gtc (SEQ ID NO:11), tcccc gct (SEQ ID NO:12) et tcccc gaa (SEQ ID NO:21), ou le complément de celle-ci, un promoteur et une séquence de codage codant pour une protéine à exprimer.

5. Vecteur selon la revendication 4, dans lequel des éléments de contrôle de transcription identiques ou différents à basse température douce consistant en une séquence de nucléotides sélectionnée parmi tcccc gcC(SEQ ID NO:2), ggggg gga (SEQ ID NO:7), gcccc gcc (SEQ ID NO:9), acccc gcc (SEQ ID NO:10), tcccc gtc (SEQ ID NO:9), acccc gcc (SEQ ID NO:10), tcccc gtc (SEQ ID NO:11), tcccc gct (SEQ ID NO:12) et tcccc gaa (SEQ ID NO:21), ou le complément de celle-ci, sont reliés.

6. Vecteur selon la revendication 5, dans lequel lesdits éléments de contrôle de transcription identiques ou différents à basse température douce sont reliés par un ou d' autre(s) nucléotide(s).

7. Vecteur selon l'une quelconque des revendications 4 à 6, qui est un vecteur mammalien.

8. Cellule mammalienne transformée par le vecteur selon l'une quelconque des revendications 4 à 7.

9. Méthode de production d'un vecteur apte à exprimer une protéine d'intérêt à de basses températures douces, la méthode comprenant la liaison ensemble et l'insertion dans un vecteur d'un élément de contrôle de transcription à basse température douce consistant en une séquence de nucléotides sélectionnée parmi tcccc gcc (SEQ ID NO:2), ggggg gga (SEQ ID NO:7), gcccc gcc (SEQ ID NO:9), acccc gcc (SEQ ID NO:10), tcccc gtc (SEQ ID NO: 9), acccc gcc (SEQ ID NO:10), tcccc gtc (SEQ ID NO:11), tcccc gct (SEQ ID NO:12) et tcccc gaa (SEQ ID NO:21), ou le complément de celle-ci, un promoteur et la séquence de codage codant pour la protéine à exprimer.

10. Méthode selon la revendication 9, dans laquelle le vecteur est un vecteur selon l'une quelconque des revendications 4 à 7.

11. Méthode de production d'une cellule hôte apte à exprimer une protéine d'intérêt à de basses températures douces, comprenant la transformation d'une cellule hôte avec un vecteur selon l'une quelconque des revendications 4 à 7.

12. Méthode selon la revendication 11, dans laquelle la cellule hôte est une cellule mammalienne.

13. Méthode d'amélioration d'un élément de contrôle de transcription à basse température douce consistant en la séquence tccccgcc ou ggggggga, comprenant:
1) synthétiser une séquence de nucléotides mutée dans laquelle un ou deux nucléotides dans l'élément de contrôle de transcription à basse température douce sont substitués par un autre nucléotide;
2) incorporer la séquence de nucléotides mutée avec un promoteur approprié dans un vecteur exprimant la chloramphénicol actyltransférase (CAT) comme un gène reporteur;
3) transformer les cellules mammaliennes par ledit vecteur;
4) cultiver les cellules à une basse température douce et 37°C, respectivement, pour exprimer le gène reporteur pour identifier la séquence de nucléotides nécessaire pour accélérer la transcription du gène reporteur à une basse température douce.
